(19) Europäisches Patentamt
European Patent Office
Office européen des brevets

(11) EP 4 653 488 A1

(12) **EUROPEAN PATENT APPLICATION**
published in accordance with Art. 153(4) EPC

(43) Date of publication:
**26.11.2025 Bulletin 2025/48**

(21) Application number: **24744893.9**

(22) Date of filing: **18.01.2024**

(51) International Patent Classification (IPC):
***C08J 3/24*** *(2006.01)* ***C08J 3/12*** *(2006.01)*
***C08B 15/00*** *(2006.01)* ***C08L 1/28*** *(2006.01)*
***C08K 5/00*** *(2006.01)* ***C08K 5/092*** *(2006.01)*
***C08K 5/07*** *(2006.01)* ***A61L 15/28*** *(2006.01)*

(52) Cooperative Patent Classification (CPC):
**A61L 15/28; C08B 15/00; C08J 3/12; C08J 3/24; C08K 5/00; C08K 5/07; C08K 5/092; C08L 1/28**

(86) International application number:
**PCT/KR2024/000879**

(87) International publication number:
**WO 2024/155113 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.01.2023 KR 20230008024**

(71) Applicant: **LG CHEM, LTD.**
**Seoul 07336 (KR)**

(72) Inventors:
- **KANG, Sun Ah**
  **Daejeon 34122 (KR)**
- **CHO, Beom Shin**
  **Daejeon 34122 (KR)**
- **KIM, Yu Min**
  **Daejeon 34122 (KR)**
- **YUN, Hae Sung**
  **Daejeon 34122 (KR)**

(74) Representative: **Plasseraud IP**
**104 Rue de Richelieu**
**CS92104**
**75080 Paris Cedex 02 (FR)**

(54) **POLYMER MATERIAL**

(57) The present application relates to a polymer material and a use thereof. The present application can provide a polymer material having excellent biodegradability and absorptivity by using a polysaccharide into which a specific functional group is introduced. The present application can also provide a use of the polymer material.

[Figure 1]

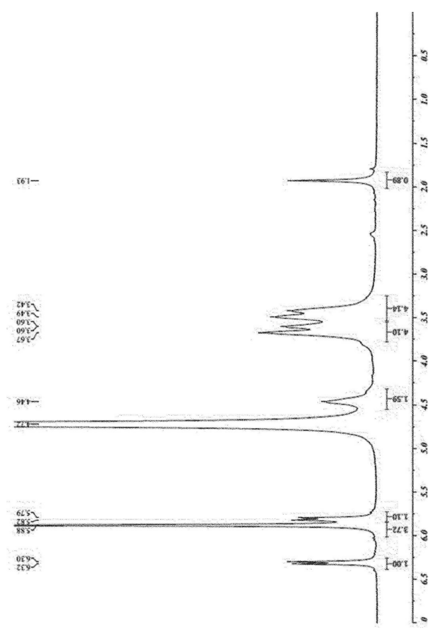

EP 4 653 488 A1

## Description

### Technical Field

This application claims the benefit of priority based on Korean Patent Application No. 10-2023-0008024 dated January 19, 2023, the disclosure of which is incorporated herein by reference in its entirety.
The present application relates to a polymer material and a use thereof.

### Background Art

A hydrogel polymer or hydrogel is generally defined as a cross-linked hydrophilic polymer.
Such a polymer can be used as a material called an SAP (Super Absorbent Polymer). The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents.
The most widely used hydrogel polymer, which is used as the SAP, is a polymer made of a vinyl-based material such as cross-linked polyacrylic acid.
Such materials are relatively inexpensive and have excellent water absorption capacity, but cause various problems because they remain semi-permanently even after disposal.
To solve such problems, there are various attempts to manufacture the SAP from so-called materials with biodegradability.
However, the materials known to date do not form the SAP with balanced physical properties. For example, the most representative physical property required for the SAP is absorption capacity, but in the SAP of a biodegradable material known to date, at least one characteristic of absorption capacity and biodegradability is not satisfactorily secured, or in some cases, both physical properties are not secured at an appropriate level.

## Disclosure

### Technical Problem

The present application is intended to provide a polymer material capable of securing simultaneously excellent absorption capacity and biodegradability, and a use thereof.

### Technical Solution

Among the physical properties mentioned in this specification, when the measurement temperature and/or pressure affects the physical property value, the relevant physical property means a physical property measured at room temperature and/or normal pressure, unless specifically mentioned otherwise.
In the present application, the term room temperature is a natural temperature without heating or cooling, which may mean, for example, any one temperature in a range of about 10°C to 30°C, or a temperature of 23°C or 25°C or so.
In the present application, the term normal pressure is a pressure when not particularly reduced or increased, which may mean a pressure of normal atmospheric pressure or so, for example, about 740 mmHg to 780 mmHg or so.
Among the physical properties mentioned in this specification, when the measurement humidity affects the physical property value, the physical property means a physical property measured at natural humidity without special adjustment at the measured temperature and pressure, unless otherwise specified.
In this specification, unless otherwise specified, the term alkyl or alkyl group means an alkyl or alkyl group with 1 to 20 carbon atoms, 1 to 16 carbon atoms, 1 to 12 carbon atoms, 1 to 8 carbon atoms, or 1 to 4 carbon atoms. Such an alkyl or alkyl group may be linear, branched, or cyclic. Such an alkyl or alkyl group may also be optionally substituted with one or more substituents.
In this specification, unless otherwise specified, the term alkylene or alkylene group means a functional group which is connected to another object by separating two hydrogen atoms from an alkane, wherein it has a structure that the two hydrogen atoms are separated from other carbon atoms of the alkane. Such an alkylene or alkylene group may be an alkylene or alkylene group with 2 to 20 carbon atoms, 2 to 16 carbon atoms, 2 to 12 carbon atoms, 2 to 8 carbon atoms, or 2 to 4 carbon atoms. Such an alkylene or alkylene group may be linear, branched, or cyclic. Such an alkylene or alkylene group may also be optionally substituted with one or more substituents.
In this specification, unless otherwise specified, the term alkylidene or alkylidene group means a functional group which is connected to another object by separating two hydrogen atoms from an alkane, wherein it has a structure that the

two hydrogen atoms are separated from one carbon atom of the alkane. Such an alkylidene or alkylidene group may be an alkylidene or alkylidene group with 1 to 20 carbon atoms, 1 to 16 carbon atoms, 1 to 12 carbon atoms, 1 to 8 carbon atoms, or 1 to 4 carbon atoms. Such an alkylidene or alkylidene group may be linear, branched, or cyclic. Such an alkylidene or alkylidene group may also be optionally substituted with one or more substituents.

In the present application, the term hydrogel polymer material means an absorbent material comprising a cross-linked polymer, and such a material may also be referred to as a hydrogel.

In this specification, the term absorbent material means a material exhibiting at least one characteristic of moisture content, centrifuge retention capacity (CRC), and absorption capacity under pressure (AUP) as defined in this specification.

For example, when the polymer material is an absorbent material, the lower limit of the moisture content of the polymer material may be 40 wt%, 45 wt%, 50 wt%, or 55 wt% or so, and the upper limit thereof may be 70 wt%, 65 wt%, or 60 wt% or so. The moisture content may also be more than or equal to, or more than any one of the above-described lower limits, or within a range of less than or equal to, or less than any one of the above-described upper limits while being more than or equal to, or more than any one of the above-described lower limits. The moisture content is the content of moisture contained in the polymer material relative to the total weight of the polymer material to be measured, which may be calculated through the weight of the polymer material containing moisture and the weight of the dried polymer material. For example, the moisture content may be calculated through the weight loss due to evaporation of moisture in the polymer material during a process of drying the polymer material in a crumb state by raising the temperature thereof through infrared heating. The drying process for measuring the moisture content may comprise raising the temperature from room temperature to about 50°C or so and then performing vacuum drying for about 6 hours or so while maintaining 50°C. The polymer material may exhibit the water content before or after cross-linking.

For example, when the polymer material is an absorbent material, the lower limit of the centrifuge retention capacity (CRC) of the polymer material according to EDANA (European Disposables and Nonwovens Association) method WSP 241.3 may be 10 g/g, 15 g/g, 20 g/g, 25 g/g, 30 g/g, 35 g/g, 40 g/g, or 45 g/g or so, and the upper limit thereof may be 60 g/g, 55 g/ g, 50 g/g, 45 g/g, 40 g/g, or 35 g/g or so. The centrifuge retention capacity (CRC) may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. The polymer material may exhibit the centrifuge retention capacity before or after cross-linking.

For example, when the polymer material is an absorbent material, the lower limit of absorption capacity under pressure (AUP) of the polymer material at 0.7 psi according to EDANA (European Disposables and Nonwovens Association) method WSP 242.3 may be 1.5 g/g, 2 g/g, 2.5 g/g, 3 g/g, 3.5 g/g, 4 *g/g,* 4.5 *g/g,* 5 *g/g,* 5.5 *g/g,* 6 g/g, 6.5 g/g, 7 g/g, 7.5 g/g, 8 *g/g, 8.5 g/g, 9* g/g, 9.5 g/g, 10 g/g, 12 g/g, 14 g/g, 16 g/g, 18 g/g, or 19 g/g or so, and the upper limit thereof may be 40 g/g, 35 g/g, 30 g/g, 20 g/g, 15 g/g, 10 g/g, 8 g/g, 6 g/g, or 4 g/g or so. The absorption capacity (AUP) may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. The polymer material may exhibit the centrifuge retention capacity before or after cross-linking.

If the polymer material exhibits at least one characteristic of the moisture content, centrifuge retention capacity, and absorption capacity under pressure as described above, the relevant material may be defined as an absorbent material. The polymer material may exhibit any one of, two or more of, or all the moisture content, centrifuge retention capacity, and absorption capacity under pressure.

As above, the polymer material can exhibit excellent absorptivity and biodegradability simultaneously.

In this specification, the biodegradable material means a material exhibiting a biodegradability degree as defined herein.

For example, if the polymer material is biodegradable, the lower limit of the biodegradability degree of the polymer material may be 60%, 62%, 64%, 66%, 68%, 70%, 72%, 74%, 76%, 78%, 80%, 82%, 84%, 86%, 88%, 90%, 92%, 94%, 96%, 98%, or 99% or so, and the upper limit thereof may be 100%, 98%, 96%, 94%, 92%, 90%, 88%, 86%, 84%, 82%, 80%, 78%, or 76% or so. The biodegradability degree may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. The biodegradability degree is measured in the manner described in the examples of this specification.

The polymer material of the present application may be simultaneously the absorbent material and the biodegradable material as described above.

The term polymer material means a material comprising a polymer. The polymer may mean a substance formed by connecting two or more unitary bodies by covalent bonds. In one example, the polymer may mean a substance comprising a structure in which two or more unitary bodies are connected by covalent bonds, and having a molecular

weight of a certain level or more. The range of the molecular weight is not limited. In one example, the lower limit of the molecular weight of the polymer may be, in terms of weight average molecular weight (Mw), 500 g/mol, 1,000 g/mol, 10,000 g/mol, 100,000 g/mol, 1,000,000 g/mol, or 10,000,000 g/mol or so, and the upper limit thereof may be 10,000,000,000 g/mol, 1,000,000,000 g/mol, 100,000,000 g/mol, or 10,000,000 g/mol or so. The weight average molecular weight may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

The weight average molecular weight is a value measured in the manner described in the examples of this specification.

In one example, the lower limit of the polymer content in the polymer material may be 55 wt%, 60 wt%, 65 wt%, 70 wt%, 75 wt%, 80 wt%, 85 wt%, 90 wt%, or 95 wt% or so, and the upper limit thereof may be 100 wt%, 98 wt%, 96 wt%, 94 wt%, 92 wt%, or 90 wt% or so. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

The polymer material of the present application may comprise a polysaccharide component.

The term polysaccharide component means a polysaccharide or a mixture of polysaccharides. In the case of a mixture of polysaccharides, the mixture may be a mixture of one type of polysaccharide (i.e., when two or more molecules of the same type of polysaccharide are present) or a mixture of two or more types of polysaccharides. Here, two or more types of polysaccharides may also mean different types of polysaccharides, and may also include polysaccharides which have the same type, but different physical properties such as a molecular weight. The polysaccharide component includes only polysaccharides.

The term polysaccharide has a meaning known in the industry. The polysaccharide generally refers to a polymer molecule in which two or more unitary bodies are linked by covalent bonds. The covalent bond connecting the unitary bodies is usually a glycosidic bond. Typically, a structure in which two unitary bodies are linked by a covalent bond such as a glycosidic bond is called a disaccharide. In this specification, unless otherwise specified, the disaccharide is also included in the category of polysaccharide.

The unitary body forming the polysaccharide may be a biomolecule composed of carbon, hydrogen, and oxygen, or composed of carbon, hydrogen, oxygen, and nitrogen. In this specification, the term biomolecule is interpreted to have the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetal glucosamine, and the like are known, but the types of biomolecules in the present application are not limited to the foregoing.

If the polymer (e.g., polysaccharide component) contained in the polymer material is in a cross-linked state and has absorption capacity, the relevant polymer material may be called the hydrogel polymer material or hydrogel herein. In one example, the polymer material may also be in a powder state formed through a grinding process or the like.

In the polymer material of the present application, the polysaccharide component may be in a cross-linked state. Here, the cross-linking means a state where two or more molecules of polysaccharide are connected by one or more chemical bonds. The cross-linking may also be formed by a chemical substance, which is referred to as a so-called cross-linking agent, other than the polysaccharide, and may be formed by a reaction between functional groups included in the polysaccharide. In this specification, when the cross-linking of the polysaccharide is performed by the reaction between functional groups included in the polysaccharide without applying other cross-linking agents, the relevant cross-linked polysaccharide may be called a self-cross-linked polysaccharide component.

In this specification, the polymer material may comprise at least a self-cross-linked polysaccharide component among the above types of cross-linked polysaccharides.

In one example, the polymer material may comprise the self-cross-linked polysaccharide component in a certain amount or more based on the total weight of the polymer material. For example, the lower limit of the ratio of the self-cross-linked polysaccharide component in the polymer material may be 55 wt%, 60 wt%, 65 wt%, 70 wt%, 75 wt%, 80 wt%, 85 wt%, 90 wt%, or 95 wt% or so, and the upper limit thereof may be 100 wt%, 98 wt%, 96 wt%, 94 wt%, 92 wt%, or 90 wt% or so. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

In another example, the ratio of the cross-linking agent cross-linking the polysaccharide component in the polymer material may be limited to a certain amount or less. Here, the cross-linking agent is a substance to form a chemical bond connecting the polysaccharide components, which means a different substance other than the polysaccharide. For example, the upper limit of the ratio of the cross-linking agent in the polymer material may be 10 wt%, 9 wt%, 8 wt%, 7 wt%,

6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof may be 0 wt% or so. The ratio of the cross-linking agent may be less than any one of the above-described upper limits, or within a range of less than or equal to, or less than any one of the above-described upper limits while being more than or equal to, or more than any one of the above-described lower limits.

In the present application, the cross-linking of the polysaccharide is performed without using a cross-linking agent or while minimizing the used amount of cross-linking agent. If a cross-linking agent other than polysaccharides is applied, the biodegradability of the polymer material may be reduced. However, since the cross-linking efficiency of the polysaccharide is generally lowered when a cross-linking agent is not used, it is not easy to obtain a polymer material with desired properties (e.g., absorption properties). In the present application, by applying a polysaccharide having one or more characteristics among the polysaccharides to be described below and/or applying a self-cross-linking reaction in a manner to be described below, it is possible to provide a polymer material having excellent physical properties (for example, absorption capacity) while having excellent biodegradation characteristics in a self-cross-linked state.

For example, the self-cross-linked polysaccharide may comprise amylose and amylopectin. As is known, amylopectin and amylose are types of polysaccharides mainly found in plants, and the starch of polysaccharides is composed of amylose and amylopectin. The amylose is composed of glucose molecules linked by $\alpha$ (1$\rightarrow$4) glycosidic bonds and has a linear chain structure, whereas the amylopectin has relatively short and highly branched chains. The amylose crystallizes relatively easily compared to the amylopectin, and the amylopectin has a relatively higher solubility in water than the amylose.

The ratio of amylose and amylopectin in the polysaccharide component and the molecular weight of the relevant polysaccharide component may be greatly related to self-cross-linking efficiency, or the absorption capacity and biodegradability degree of the polymer material.

As the ratio of amylopectin in the polysaccharide component decreases and the ratio of amylose increases, the biodegradability degree of the material increases, but the absorption capacity tends to decrease somewhat. In addition, as the molecular weight of the polysaccharide component increases, the biodegradability degree of the material decreases, but the absorption capacity tends to increase.

For example, the polymer material or polysaccharide component may have F in a predetermined range according to Equation 1 below.

[Equation 1]

$$F = Log(Mw \times \frac{P}{P+M})$$

In Equation 1, P is the ratio of the amylopectin in the polysaccharide component, M is the ratio of the amylose in the polysaccharide component, and Mw is the weight average molecular weight of the polysaccharide component.

F in Equation 1 is a factor representing the amounts of amylose and amylopectin in the polysaccharide component, and the molecular weight of the polysaccharide component. For example, the value of F above increases as the molecular weight of the polysaccharide component increases and/or the ratio of amylopectin in the polysaccharide component increases.

The polysaccharide component in which the ratio of amylose and amylopectin, and the molecular weight are controlled may be efficiently cross-linked to form a material with excellent absorption capacity and biodegradability degree.

In Equation 1, P and M are the ratios of amylopectin and amylose measured in the manner described in the examples of this specification, and the unit thereof is %.

In Equation 1, Mw is the weight average molecular weight of the polysaccharide component, which is measured in the manner described in the examples of this specification, and the unit thereof is g/mol.

The lower limit of F in Equation 1 may be 4, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, or 8.2 or so, and the upper limit thereof may be 20, 18, 16, 14, 12, 10, 9, 8, 7, 6, or 5 or so. The F may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

Within such a range, the polysaccharide component may be efficiently cross-linked to form a polymer material with excellent absorption capacity and biodegradability.

In Equation 1, the ranges of Mw, M, and P may be adjusted to an appropriate level.

For example, the lower limit of Mw in Equation 1 may be 200,000 g/mol, 250,000 g/mol, 300,000 g/mol, 350,000 g/mol, 400,000 g/mol, 450,000 g/mol, 500,000 g/mol, 550,000 g/mol, 600,000 g/mol, 650,000 g/mol, 700,000 g/mol,

750,000 g/mol, 800,000 g/mol, 850,000 g/mol, 900,000 g/mol, 950,000 g/mol, 1,000,000 g/mol, 1,500,000 g/mol, 2,000,000 g/mol, 2,500,000 g/mol, 3,000,000 g/mol, 3,500,000 g/mol, 4,000,000 g/mol, 4,500,000 g/mol, 5,000,000 g/mol, 5,500,000 g/mol, 6,000,000 g/mol, 6,500,000 g/mol, 7,000,000 g/mol, 7,500,000 g/mol, 8,000,000 g/mol, 8,500,000 g/mol, 9,000,000 g/mol, 9,500,000 g/mol, 10,000,000 g/mol, 20,000,000 g/mol, 30,000,000 g/mol, 40,000,000 g/mol, 50,000,000 g/mol, 60,000,000 g/mol, 70,000,000 g/mol, 80,000,000 g/mol, 90,000,000 g/mol, 100,000,000 g/mol, 110,000,000 g/mol, 120,000,000 g/mol, 130,000,000 g/mol, 140,000,000 g/mol, 150,000,000 g/mol, 160,000,000 g/mol, 170,000,000 g/mol, or 180,000,000 g/mol or so, and the upper limit thereof may be 10,000,000,000 g/mol, 5,000,000,000 g/mol, 1,000,000,000 /mol, 900,000,000 /mol, 800,000,000 /mol, 700,000,000 /mol, 600,000,000 /mol, 500,000,000 /mol, 400,000,000 /mol, 300,000,000 /mol, 200,000,000 /mol, 150,000,000 /mol, 100,000,000 /mol, 90,000,000 /mol, 80,000,000 /mol, 70,000,000 /mol, 60,000,000 /mol, 50,000,000 /mol, 40,000,000 /mol, 30,000,000 /mol, 20,000,000 /mol, 10,000,000 /mol, 9,000,000 /mol, 8,000,000 /mol, 7,000,000 /mol, 6,000,000 /mol, 5,000,000 /mol, 4,000,000 /mol, 3,000,000 /mol, 2,000,000 /mol, 1,000,000 /mol, 900,000 /mol, 800,000 /mol, 700,000 /mol, 600,000 /mol, or 500,000 /mol or so. The Mw may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

The lower limit of P in Equation 1 may be 5%, 10%, 15%, 20%, 25%, 30%, 35%, 40%, 45%, 50%, 55%, 60%, 65%, 70%, 75%, or 80%, and the upper limit thereof may be 95%, 90%, 85%, 80%, 75%, 70%, 65%, 60%, 55%, 50%, 45%, 40%, 35 %, 30%, 35%, or 20% or so. The P may be more than or equal to, or more than any one of the above-described lower limits, less than or equal to, or less than any one of the above-described upper limits, or within a range of less than or equal to, or less than any one of the above-described upper limits while being more than or equal to, or more than any one of the above-described lower limits.

The lower limit of M in Equation 1 may be 5%, 10%, 15%, 20%, 25%, 30%, 35%, 40%, 45%, 50%, 55%, 60%, 65%, 70%, 75%, or 80%, and the upper limit thereof may be 95%, 90%, 85%, 80%, 75%, 70%, 65%, 60%, 55%, 50%, 45%, 40%, 35 %, 30%, 35%, or 20% or so. The M may be more than or equal to, or more than any one of the above-described lower limits, less than or equal to, or less than any one of the above-described upper limits, or within a range of less than or equal to, or less than any one of the above-described upper limits while being more than or equal to, or more than any one of the above-described lower limits.

In one example, the sum of M and P above in the polysaccharide component may be 100%.

In one example, when the polysaccharide of the polysaccharide component is a modified polysaccharide to be described below, that is, a polysaccharide into which an acidic group such as a carboxyl group is introduced by maleation or carboxyalkylation, the functional group introduced into the modified polysaccharide may be introduced into either amylose or amylopectin, or may be introduced into both. The reaction of introducing the functional group, which is described below, can occur in both amylose and amylopectin, but more efficient modification may be possible when the content of amylopectin is greater than the content of amylose.

As the polysaccharide component, an appropriate type may be selected and used among the above-mentioned polysaccharides if it contains at least polysaccharides including amylose and amylopectin. One, or two or more of known polysaccharides are combined to form a polysaccharide component so that F in Equation 1 is 4 or more, which may be applied to the polymer material.

In the present application, the self-cross-linking of the polysaccharide may be performed using a so-called acidic polysaccharide. As is known, the acidic polysaccharide is a polysaccharide having this acidic group, where an example of the acidic group may include a carboxylic group, a phosphate group, a phosphite group, and/or a sulfuric acid ester group (sulfuric ester group), or their salts.

A substitution degree of the acidic polysaccharide or acidic polysaccharide component may be adjusted for appropriate self-cross-linking. The substitution degree is an indicator indicating the amount of the acidic group present in the acidic polysaccharide or polysaccharide component, where such a substitution degree may be, for example, a value obtained before the polysaccharide or polysaccharide component implements the cross-linked structure.

For example, the lower limit of the substitution degree may be 0.4, 0.45, 0.5, 0.6, 0.65, 0.7, 0.75, 0.8, 0.85, 0.9, 0.95, or 1 or so, and the upper limit thereof may be 2.5, 2, 1.5, 1.1, 1.05, 1, 0.95, 0.9, 0.85, 0.8, 0.75, 0.7, 0.65, or 0.6 or so. The substitution degree may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. Within the range of the substitution degree, the cross-linking of the polysaccharide may proceed effectively, and the physical properties (for example, absorption capacity and/or biodegradability, etc.) of the resulting polymer material may be stably secured. When the substitution degree is high, the number of acidic groups, which are hydrophilic functional groups, increases in the polysaccharide or polysaccharide component, so that it may be advantageous in terms of absorption characteristics, but too many acidic groups excessively promote the cross-linking reaction, whereby absorption characteristics after cross-linking may be lowered. Therefore, considering these points, it is possible

to select an appropriate substitution degree.

In this specification, the substitution degree is an indicator of the degree of acidic group present in the polysaccharide or polysaccharide component, which is, for example, a value representing to what extent the functional group such as a hydroxy group present in each unitary body contained in the polysaccharide or polysaccharide component is substituted with a predetermined acidic group (for example, the carboxyl group) and is an average value for each unitary body present in the polysaccharide. For example, if the unitary body is a grape sugar (glucose) unit, there are three hydroxy groups in the relevant unit before modification, and thus, if all the hydroxy groups are replaced with the functional groups of Formula 1 above, the substitution degree for the relevant unit is 3. However, the substitution degree of the polysaccharide is the average value of the substitution degree of each unitary body present in the relevant polysaccharide, so that for example, in a polysaccharide containing 5 grape sugar (glucose) units, if the substitution degree of each unit is 1, 0, 2, 3, and 1, the substitution degree of the polysaccharide becomes 1.4, which is the average value. This substitution degree may be identified through $^1$H NMR analysis of the polysaccharide. That is, since the hydroxyl groups and substituted functional groups present in the polysaccharide can be quantified through the $^1$H NMR analysis, the substitution degree can be identified, and the substitution degree can be calculated in consideration of the $^1$H NMR analysis results of the polysaccharide before modification, if necessary. In this way, the method of quantifying functional groups through $^1$H NMR analysis is known.

In the present application, the self-cross-linking of the polysaccharide may be performed, for example, using a polysaccharide or polysaccharide component having a carboxyl group as an acidic group among such acidic polysaccharides or acidic polysaccharide components. Since the polysaccharide itself contains hydroxy groups, the hydroxy groups contained in one molecule of polysaccharide may be subjected to an esterification reaction with the carboxyl groups contained in the acidic polysaccharide to perform the self-cross-linking.

The type of polysaccharide component having the carboxyl group is not particularly limited. For example, the self-cross-linking may be performed by applying polysaccharides having carboxyl groups on their own, such as so-called CMC (carboxylmethyl cellulose), or polysaccharides to which carboxyl groups are introduced through processes such as maleation or carboxyalkylation.

The self-cross-linked polysaccharide component may comprise polymer chains (i.e., polysaccharide chains to be cross-linked) containing monosaccharide units linked by glycosidic bonds, and cross-linking bonds linking the polymer chains.

At this time, the cross-linking bond may be connected to the monosaccharide unit.

Here, the cross-linking bond may be a bond represented by Formula 1 below.

[Formula 1]

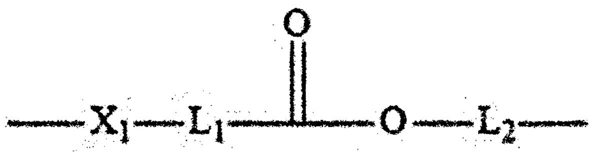

In Formula 1, $X_1$ is an oxygen atom or $NR_{11}$, where $R_{11}$ is a hydrogen atom, an alkyl group or an alkylcarbonyl group, and $L_1$ is an alkylene group, an alkylidene group, or a bond of Formula 2 below, and $L_2$ is represented by a single bond or $-(CH_2)-O-$.

[Formula 2]

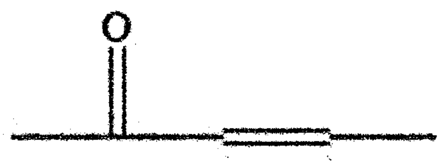

In Formula 1, $X_1$ shown on the leftmost side may be directly connected to the monosaccharide unit of the polymer chain.

In the case where $X_1$ of Formula 1 is an oxygen atom, it is the case where starch or the like as the polysaccharide is applied to the cross-linking, and in the case of $NR_{11}$, it is the case where chitosan or chitin, and the like is applied to the

cross-linking.

The alkyl group of $R_{11}$ may be an alkyl group with 1 to 20 carbon atoms, 1 to 16 carbon atoms, 1 to 12 carbon atoms, 1 to 8 carbon atoms, or 1 to 4 carbon atoms, or a methyl group, and such an alkyl group may be linear, branched, or cyclic, which may optionally be substituted with one or more substituents.

In Formula 1, when $L_2$ is a single bond, the $L_2$ does not exist. That is, in Formula 1, when $L_2$ exists, $L_2$ may be directly connected to the monosaccharide unit of the polymer chain, and when $L_2$ is a single bond, the oxygen atom on the left side of $L_2$ in Formula 2 may be directly connected to the monosaccharide unit.

In the case where the self-cross-linking of the polysaccharide is performed by a polysaccharide having a carboxyl group on its own, such as CMC (carboxylmethyl cellulose), or a polysaccharide having a carboxyl group introduced by carboxyalkylation or the like, $L_1$ in Formula 1 may usually be an alkylene group or an alkylidene group. In Formula 1, when $L_1$ is a functional group of Formula 2, it is the case where the self-cross-linking is performed by a carboxyl group introduced by so-called maleation or the like.

In Formula 1, when $L_1$ is Formula 2, any one of the carbon atoms of the carbonyl group in Formula 2 is connected to $X_1$ on the left side of $L_1$ in Formula 1, and the carbon atom on the right side of the carbon-carbon double bond is connected to the carbon atom of the carbonyl group on the right side of $L_1$.

One or more bonds of Formula 1 above may exist within the self-cross-linked polysaccharide component.

The type of the monosaccharide unit is not particularly limited, which may be typically a monosaccharide unit constituting a polysaccharide. For example, such a monosaccharide unit may be a glucose unit, a glucosamine unit, an N-acetylglucosamine unit, and the like.

Such a monosaccharide unit usually contains a ring structure containing carbon atoms and oxygen atoms as ring constituent atoms. The ring structure of the monosaccharide unit is usually a hexa-cyclic ring structure (in the case where the ring atoms include only 5 carbon atoms and 1 oxygen atom), but may also have a ring structure of 6 rings or more. When the ring structure is six rings or more, the ring atoms may be carbon atoms, or heteroatoms such as oxygen or nitrogen atoms.

In the case of the self-cross-linking structure, the bond of Formula 1 above may be directly connected to the carbon atom of the ring structure of the monosaccharide unit, or may be connected via a methylene group ($-CH_2-$).

At least one of or both the leftmost $X_1$ and the rightmost oxygen atom (if $L_2$ is a single bond, it means the oxygen atom connected to the right side of the carbonyl group, and if it is not a single bond, it means the oxygen atom of $-(CH_2)-O-$) in Formula 1 may be directly connected to the carbon atom of the ring structure, or may be connected via the methylene group ($-CH_2-$).

Here, the matter of being connected via the methylene group ($-CH_2-$) is the case where only the methylene group ($-CH_2-$) exists between the leftmost $X_1$ of Formula 1 above or the rightmost oxygen atom (if $L_2$ is a single bond, it means the oxygen atom connected to the right side of the carbonyl group, and if it is not a single bond, it means the oxygen atom of $-(CH_2)-O-$) of Formula 1 and the carbon atom of the ring structure.

In this case, more specifically, the polysaccharide component may contain a unit represented by Formula 3 below.

[Formula 3]

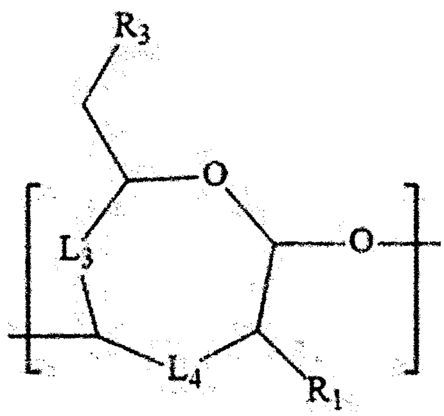

In Formula 3, $R_1$ is a hydroxy group, an amino group, $-L_5-C(=O)-OH$, $-L_5-C(=O)-O^-$, or a functional group of Formula 4 below, $R_3$ is a hydroxy group, $-L_5-C(=O)-OH$, $-L_5-C(=O)-O^-$ or a functional group of Formula 4 below, and one of $L_3$ and $L_4$ is a single bond, the other is $CHR_2$, where $R_2$ is a hydroxy group, $-L_5-C(=O)-OH$, $-L_5-C(=O)-O^-$ or a functional group of Formula 4 below, and $L_5$ is an alkylene group or an alkylidene group, but any one of $R_1$ to $R_3$ is the oxygen atom

(excluding the oxygen atom present in the carbonyl group) of the bond of Formula 1 above.

[Formula 4]

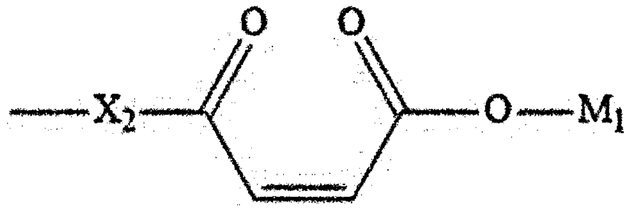

In Formula 4, $X_2$ is an oxygen atom or $NR_{11}$, where $R_{11}$ is a hydrogen atom, an alkyl group or an alkylcarbonyl group, $M_1$ is hydrogen or a metal, and when $M_1$ is the metal, the bond of O-$M_1$ above is an ionic bond.

In Formula 3, the case where $R_1$ is an amino group is the case where the unit is a glucosamine unit or N-acetylglucosamine unit. The amino group at this time may be optionally substituted with one or more substituents. At this time, the substituent may be exemplified by an alkyl group or an alkylcarbonyl group. In this case, the alkyl group may be an alkyl group with 1 to 20 carbon atoms, 1 to 16 carbon atoms, 1 to 12 carbon atoms, 1 to 8 carbon atoms, or 1 to 4 carbon atoms, or a methyl group, where such an alkyl group may be linear, branched, or cyclic. and may also be optionally substituted with one or more substituents.

The functional group of $-L_5-C(=O)-OH$ or $-L_5-C(=O)-O^-$ in Formula 3 may be, for example, a carboxyl group introduced by carboxyalkylation, or a functional group in which the carboxyl group is ionized, and the like, and Formula 4 is a functional group introduced by maleation.

Such functional groups are introduced for forming cross-linking bonds by participating in the above-described self-cross-linking reaction, but all the introduced functional groups may not participate in the cross-linking reaction, and in this case, some functional groups may remain.

Here, the fact that any one of $L_3$ and $L_4$ is a single bond means that any one of $L_3$ and $L_4$ does not exist. For example, when $L_3$ is not present, the carbon atoms connected to the left and right of $L_3$ in Formula 3 are directly connected, and when $L_4$ is not present, the carbon atoms connected to the left and right of $L_4$ in Formula 3 are directly connected.

Here, the fact that the other of $L_3$ and $L_4$ is $CHR_2$ means that in Formula 3, any one of $L_3$ and $L_4$ is a carbon atom, and the substituent $R_2$ is substituted on the carbon atom.

The fact that in Formula 3, any one of $R_1$ to $R_3$ is the oxygen atom (excluding the oxygen atom present in the carbonyl group) of the bond of Formula 1 means that any one of $R_1$ to $R_3$ is the oxygen atom of the bond of Formula 1 connecting the polysaccharide, where the oxygen atom means any one of the leftmost oxygen atom and the rightmost oxygen atom in Formula 1 (if $L_2$ is a single bond, it means the oxygen atom connected to the right of the carbonyl group, and if it is not a single bond, it means the oxygen atom of $-(CH_2)-O-$).

In the case where $X_2$ of Formula 4 is an oxygen atom, it is the case where starch or the like as the polysaccharide is maleated, and in the case of $NR_{11}$, it is the case where chitosan or chitin, and the like as the polysaccharide is maleated. The alkyl group of $R_{11}$ may be an alkyl group with 1 to 20 carbon atoms, 1 to 16 carbon atoms, 1 to 12 carbon atoms, 1 to 8 carbon atoms, or 1 to 4 carbon atoms, or a methyl group, and such an alkyl group may be linear, branched, or cyclic, and may also be optionally substituted with one or more substituents.

As described above, the self-cross-linking structure may be implemented by performing an esterification reaction between acidic polysaccharides, especially, polysaccharides having a carboxyl group.

As the acidic polysaccharides, polysaccharides having a carboxyl group on their own, such as CMC (carboxylmethyl cellulose), may be used, or polysaccharides in which a carboxyl group is introduced through a process such as maleation or carboxyalkylation may be used, where to achieve the above-mentioned substitution degree and for efficient self-cross-linking, polysaccharides into which a carboxyl group has been introduced through a denaturation process may be used.

The method of introducing a carboxyl group into a polysaccharide is not particularly limited. For example, to introduce a functional group such as Formula 4, a so-called maleation process may be performed. Such a process is a process of reacting a polysaccharide with an unsaturated dicarboxylic acid or its anhydride to replace the hydroxy group present in the unitary body of the polymer with the functional group, where an example of the dicarboxylic acid or its anhydride may be exemplified by maleic acid or maleic anhydride, and the like, but is not limited thereto, and salts of the maleic acid, and the like may also be applied. The method of performing the maleation process is known.

The carboxyalkylation process may be performed by reacting the polysaccharide with an alkanoic acid or haloalkanoic acid or a salt of the alkanoic acid or haloalkanoic acid. As is known, the alkanoic acid is an aliphatic acid derived from an alkane, and the haloalkano acid means one in which at least one of hydrogen atoms of the alkanoic acid is

replaced with a halogen atom (e.g., chlorine, fluorine, or bromine, etc.). In this specification, the alkanoic acid, haloalkanoic acid, salt of alkanoic acid, and/or salt of haloalkanoic acid applied in the carboxyalkylation process may be referred to as a treatment agent.

As the alkano acid or haloalkano acid used as the treatment agent, an alkanoic acid or haloalkano acid with 1 to 20 carbon atoms, 1 to 16 carbon atoms, 1 to 12 carbon atoms, 1 to 8 carbon atoms, or 1 to 4 carbon atoms may be used, and acetic acid or chloroacetic acid may be typically used.

In addition, here, the salt of the haloalkanoic acid or alkanoic acid may be an alkali metal salt or alkali earth metal salt of the haloalkanoic acid or alkanoic acid having the same number of carbon atoms as above.

An acidic group (carboxyl group) may be introduced into the polysaccharide by reacting the treatment agent with the polysaccharide under appropriate conditions.

The process of introducing the acidic group may be performed according to a known method, and if necessary, an additional process may be performed or the conditions of the process may be adjusted for efficient progress of the carboxyalkylation process.

For example, the above process may be performed on a mixture in which the treating agent and a hydroxide are dispersed in a solvent. Here, as the solvent, for example, an aqueous solvent such as water may be used. Here, as the water, tap water, distilled water, deionized water, or purified water, and the like may be used. Here, as the hydroxide, ammonium hydroxide or a metal hydroxide may be used, and as the metal hydroxide, sodium hydroxide, potassium hydroxide, or lithium hydroxide, and the like may be used, without being limited thereto.

It is expected that in the reaction to the mixture, for example, a gelatinization reaction of polysaccharides first proceeds within the mixture, and subsequently introduction of carboxyl groups by the treatment agent proceeds.

The reaction may be performed, for example, while the torque of the reactor where the mixture exists after the gelatinization becomes a certain level. In this way, the introduction of a suitable carboxyl group proceeds.

For example, if the mixture is mixed in a mixer, such as an internal mixer, capable of mixing the components by rotation, the gelatinization proceeds, where heat energy is generated inside the reactor by the load caused by the gelatinization during a process that the gelatinization proceeds, whereby the torque and temperature increase. Typically, a section where the torque and temperature within the reactor increase to a certain level, and then remain constant occurs, where the time point when the section occurs is usually regarded as the time point when the gelatinization is completed. By maintaining the torque at an appropriate level at the gelatinization completion time point and/or the time point afterward to have a desired substitution degree, and the like, it is possible to obtain a polysaccharide capable of efficiently generating the self-cross-linking.

In one example, the lower limit of the torque in the reactor at the gelatinization completion time point and/or after the gelatinization completion time point may be 5 Nm, 5.5 Nm, 7 Nm, 7.5 Nm, or 8 Nm or so, and the upper limit thereof may be 20 Nm, 19 Nm, 18 Nm, 16 Nm, 15 Nm, 14 Nm, 13 Nm, 12 Nm, 11 Nm, 10 Nm, or 9 Nm or so. The torque may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. By maintaining the torque in the above range, it is possible to prevent excessive evaporation of the solvent, and it is possible to maintain workability stably, while maintaining the substitution efficiency of the carboxyl group in the desired range.

To maintain the torque as above, the ratio of solvent in the mixture may be controlled. For example, the lower limit of the ratio of the solvent in the mixture may be 0.45 times, 0.5 times, 0.6 times, or 0.7 times or so the weight of the polysaccharide present in the mixture, and the upper limit thereof may be 0.75 times, 0.74 times, 0.73 times, or 0.72 times or so the weight of the polysaccharide present in the mixture. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. By maintaining the ratio of the solvent within the above range, it is possible to prevent excessive evaporation of the solvent, and it is possible to maintain the torque capable of stably maintaining workability, while maintaining the substitution efficiency of the carboxyl group in the desired range.

In the above reaction process, it may be appropriate to use substantially only the above-described aqueous solvent (e.g., water) as the solvent. Generally, as the solvent for carboxyalkylation, alcohol, ketone, 1,4-dioxane, dimethylformamide, or dimethyl sulfoxide, and the like may also be applied in addition to the aqueous solvent, but when such a solvent is applied, the reaction may not proceed as desired.

The mixture may contain substantially no solvent other than the aqueous solvent (e.g., water). At this time, the fact that it contains substantially no solvent may mean the case where the upper limit of the content of solvents other than the aqueous solvent (e.g., water) in the mixture is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be less than any one of the above-described upper limits, or within a range of less than or equal to, or less than any one of the above-described upper limits while being more than or equal to, or more than any one of the above-described lower limits.

The ratios of the hydroxide and treating agent in the mixture may also be controlled.

For example, the lower limit of the ratio of the hydroxide in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be less than or equal to, or less than any one of the above-described upper limits, or within a range of less than or equal to, or less than any one of the above-described upper limits while being more than or equal to, or more than any one of the above-described lower limits. Here, the equivalent can be obtained by an equation A/B, wherein A is the mole number of the hydroxide present in the mixture, and B is a value calculated as C/162.14, where C is the weight (unit: g) of the polysaccharide in the mixture. Here, 162.14 is the molar mass (g/mol) of anhydroglucose unit. Typically, the polysaccharide contains the anhydroglucose unit or a derivative thereof, or a unit with a similar molar mass. Therefore, in the present application, if the used amount of polysaccharide for obtaining the above equivalent is representatively applied by the equation C/162.14, the reaction may proceed according to the purpose by specifying the equivalent according to such an applying manner in the above range. By maintaining the used amount of hydroxide in the above range, it is possible to stably maintain the reaction efficiency and workability while maintaining the substitution efficiency of carboxyl groups in the desired range, and it is possible to suppress unnecessary side reactions, and the like.

In addition, the lower limit of the ratio of the treatment agent in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be less than or equal to, or less than any one of the above-described upper limits, or within a range of less than or equal to, or less than any one of the above-described upper limits while being more than or equal to, or more than any one of the above-described lower limits. Here, the equivalent can be obtained by an equation D/B, wherein D is the mole number of the treatment agent present in the mixture, and B is the same as the equation for calculating the equivalent of the hydroxide. By maintaining the used amount of the treatment agent in the above range, it is possible to stably maintain the reaction efficiency and workability while maintaining the substitution efficiency of carboxyl groups in the desired range, and it is possible to suppress unnecessary side reactions, and the like.

The lower limit of the ratio (A/B) of the mole number (A) of the hydroxide and the mole number (D) of the treatment agent in the mixture may be 0.5, 0.6, 0.7, 0.8, 0.9, or 0.95 or so, and the upper limit thereof may be 1.5, 1.4, 1.3, 1.2, 1.1, or 1.05 or so. The ratio may be less than or equal to, or less than any one of the above-described upper limits, or within a range of less than or equal to, or less than any one of the above-described upper limits while being more than or equal to, or more than any one of the above-described lower limits. By maintaining the above ratio, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

In the above reaction, the mixture may be present in a predetermined ratio in a reactor where the reaction occurs. For example, the lower limit of the ratio of the volume of the mixture in the reactor based on the total volume of the reactor may be 70%, 75%, 76%, or 77% or so, and the upper limit thereof may be 95%, 94%, 93%, or 92% or so. The ratio may be less than or equal to, or less than any one of the above-described upper limits, or within a range of less than or equal to, or less than any one of the above-described upper limits while being more than or equal to, or more than any one of the above-described lower limits. By maintaining the above ratio, it is possible to stably maintain torque in the reactor, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

In the above reaction, the temperature in the reactor at the gelatinization completion time point and/or after the time point may also be maintained at a certain level. For example, the lower limit of the temperature may be 90°C, 92°C, 94°C, or 96°C or so, and the upper limit thereof may be 110°C, 105°C, 100°C, 99°C, 98°C, or 97°C or so. The temperature may be less than or equal to, or less than any one of the above-described upper limits, or within a range of less than or equal to, or less than any one of the above-described upper limits while being more than or equal to, or more than any one of the above-described lower limits. By maintaining the above temperature, it is possible to stably maintain torque in the reactor, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

As described above, the carboxyalkylation may be performed using a reactor capable of mixing the mixture by rotation. The specific type of reactor is not particularly limited, and for example, an internal mixer such as a two-roll mixer, a Banbury mixer and an intermix mixer may be used. The torque and temperature may also be measured through sensors mounted on such a mixer.

The mixing time is not particularly limited, which may be controlled to a level where the desired gelatinization and carboxyalkylation may be achieved. For example, the lower limit of the mixing time may be 5 minutes, 7 minutes, 9 minutes, or 10 minutes or so, and the upper limit thereof may be 60 minutes, 55 minutes, 50 minutes, 45 minutes, 40 minutes, 35

minutes, 30 minutes, 25 minutes, 20 minutes, 15 minutes, 14 minutes, 13 minutes, 12 minutes, 11 minutes, or 10 minutes or so. The mixing time may be less than or equal to, or less than any one of the above-described upper limits, or within a range of less than or equal to, or less than any one of the above-described upper limits while being more than or equal to, or more than any one of the above-described lower limits.

The rotating speed of the mixing may be controlled to maintain the desired torque and/or temperature. The lower limit of the rotating speed may be, for example, 30 rpm, 35 rpm, 40 rpm, 45 rpm, or 50 rpm or so, and the upper limit thereof may be 100 rpm, 95 rpm, 90 rpm, 85 rpm, 80 rpm, 75 rpm, 70 rpm, 65 rpm, 60 rpm, 55 rpm, or 50 rpm or so. The rotating speed may be less than or equal to, or less than any one of the above-described upper limits, or within a range of less than or equal to, or less than any one of the above-described upper limits while being more than or equal to, or more than any one of the above-described lower limits.

Through the above process, the carboxyalkylation progresses, and it is possible to obtain the desired polysaccharide.

Immediately after introducing a carboxyl group into the polysaccharide through the reaction, it is also possible to perform the self-cross-linking process, and if necessary, after recovering the polysaccharide once, it is possible to perform the self-cross-linking process.

Here, the recovering of the polysaccharide may comprise a process of dissolving the reactant resulting from the reaction in water and precipitating it using an organic solvent such as alcohol, and in addition to this, various methods may be applied.

After obtaining the acidic polysaccharide in the above manner, it may be self-cross-linked. The method of performing the self-cross-linking is not particularly limited, but for efficient self-cross-linking, it may be performed by a method of dispersing the acidic polysaccharide in a solvent, and then maintaining the pH within a predetermined range.

In the above process, the above-described aqueous solvent, for example, water may also be used as the solvent, and tap water, distilled water, deionized water, or purified water, and the like may be used as the water.

Even upon the self-cross-linking, it may be appropriate to substantially use only the aqueous solvent (e.g., water) as the solvent. Therefore, the solvent used upon the self-cross-linking may not substantially contain other solvents rather than the aqueous solvent (e.g., water). At this time, the matter that other solvents are not substantially included may mean the case where the upper limit of the content of other solvents rather than the aqueous solvent (for example, water) in the solvent is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be less than or equal to, or less than any one of the above-described upper limits, or within a range of less than or equal to, or less than any one of the above-described upper limits while being more than or equal to, or more than any one of the above-described lower limits.

The amount of the applied solvent n the above process may be an amount of 5 times to 15 times the weight of the applied polysaccharide. The dissolution of the polysaccharide into the solvent may be performed under room temperature and normal pressure conditions, but is not limited thereto.

The self-cross-linking may proceed by dissolving the polysaccharide in the solvent and maintaining the pH at a constant level. If necessary, additional processes, such as a stirring process, capable of promoting the self-cross-linking may also be performed.

The lower limit of pH maintained in the above process may be 4, 4.5, 5, 5.5, or 6 or so, and the upper limit thereof may be 10, 9.5, 9, 8.5, 8, 7.5, 7, or 6.5 or so. The pH may be less than or equal to, or less than any one of the above-described upper limits, or within a range of less than or equal to, or less than any one of the above-described upper limits while being more than or equal to, or more than any one of the above-described lower limits. By maintaining this pH range, it is possible to effectively perform the desired self-cross-linking.

The method of maintaining pH in the above range is not particularly limited, and if the pH in the above range is achieved by adding an acidic polysaccharide, the self-cross-linking may proceed in that state, and if the desired pH is not achieved, the pH may be adjusted by adding an appropriate acid or base in consideration of the desired pH. At this time, for example, the hydroxide applied in the carboxyalkylation may be used as the base, and hydrochloric acid or sulfuric acid, and the like may be used as the acid, without being limited thereto.

In the above reaction process, a catalyst may be added as needed. For example, an ester catalyst promoting the reaction of carboxyl groups and hydroxy groups may be added. As such a catalyst, 4-methylaminopyridine, magnesium acetate, tetra-n-butyl titanate, lead acetate, sodium acetate, potassium acetate, antimony trioxide and/or N-methylimidazole, and the like may be used, without being limited thereto. The catalyst may be added in a catalytic amount and, for example, may be used in a ratio within a range of 0.1 mol to 5 mol relative to 1 mol of the polysaccharide applied to the reaction. The lower limit of the catalyst usage ratio may be 0.1 mol, 0.5 mol, 1 mol, or 2 mol or so, and the upper limit thereof may be 5 mol, 4.5 mol, 4 mol, or 3.5 mol or so. The ratio may be less than or equal to, or less than any one of the above-described upper limits, or within a range of less than or equal to, or less than any one of the above-described upper limits while being more than or equal to, or more than any one of the above-described lower limits.

The reaction may also be performed in the presence of a heat stabilizer, if necessary. As the applicable heat stabilizer, an organic or inorganic phosphorus compound such as phosphoric acid, an organic ester of phosphoric acid,

phosphorous acid or an organic ester of phosphorous acid may be used, and for example, phosphoric acid, alkyl phosphate or aryl phosphate, and the like, which is commercially known as the heat stabilizer, may be used.

In addition, the reaction may also be performed in the presence of additives such as thickeners, plasticizers, preservation stabilizers, and/or antioxidants, if necessary.

The cross-linking reaction may be performed at a predetermined temperature. For example, the lower limit of the temperature at which the reaction proceeds may be 80°C, 85°C, 90°C, 95°C, 100°C, 105°C, 110°C, 115°C, or 120°C or so, and the upper limit thereof may be 300°C, 280°C, 260°C, 240°C, 220°C, 200°C, 180°C, 160°C, 140°C, 130°C, 125°C, or 125°C or so. The temperature may be less than or equal to, or less than any one of the above-described upper limits, or within a range of less than or equal to, or less than any one of the above-described upper limits while being more than or equal to, or more than any one of the above-described lower limits. Such a reaction temperature may be achieved by a method, such as hot air supply, infrared irradiation, microwave irradiation, or ultraviolet irradiation.

The time for the reaction to proceed is not particularly limited. For example, the lower limit of the reaction time may be 20 minutes, 40 minutes, 60 minutes, 80 minutes, 100 minutes, or 120 minutes or so, and the upper limit thereof may be 500 minutes, 480 minutes, 460 minutes, 440 minutes, 420 minutes, 400 minutes, 380 minutes, 360 minutes, 340 minutes, 320 minutes, 300 minutes, 280 minutes, 260 minutes, 240 minutes, 220 minutes, 200 minutes, or 180 minutes or so. The time may be less than or equal to, or less than any one of the above-described upper limits, or within a range of less than or equal to, or less than any one of the above-described upper limits while being more than or equal to, or more than any one of the above-described lower limits.

The desired self-cross-linked polysaccharide component can be obtained by the above method.

The polymer material may further comprise, together with the self-cross-linked polysaccharide component, a cross-linking agent reacting with the polysaccharide component. Such a treating agent may be introduced by reacting the self-cross-linked polysaccharide component with the cross-linking agent. That is, the polymer material of the present application may be provided in a state where two or more molecules of polysaccharide are cross-linked by the above-described self-cross-linking and then further cross-linked by applying the cross-linking agent. This further cross-linking is introduced for improving the gel strength of the self-cross-linked polysaccharide component and improving the absorption capacity under pressure (AUP) and/or saline flow conductivity.

The additional cross-linking agent may also be introduced, for example, by subjecting the self-cross-linked polysaccharide component to a treatment such as grinding to powder it and reacting the surface of the relevant powder with the cross-linking agent. In this case, the polymer material may comprise the self-cross-linked polysaccharide component in a particle shape and the cross-linking agent bound to the surface of the particle. In such a case, the size of the particles is not particularly limited, and the particles may be controlled to an appropriate size depending on the applied use.

Such a cross-linking agent may have two or more functional groups capable of reacting with the functional groups (hydroxy group, amino group, or carboxyl group, etc.) of the polysaccharide component. The cross-linking agent may have 2 to 10, 2 to 9, 2 to 8, 2 to 7, 2 to 6, 2 to 5, 2 to 4, or 2 to 3, or 2 or 3 functional groups capable of reacting with the functional groups (hydroxy group, amino group, or carboxyl group, etc.) of the polysaccharide component.

As the type of the applicable cross-linking agent, one or more selected from the group consisting of a polyfunctional epoxy compound, an epoxy silane compound, an amino silane compound, epichlorohydrin, an aldehyde compound such as formaldehyde or glutaraldehyde, an acyl chloride, a carbonate, a diamine, a diol, carbon disulfide, phosphoryl chloride, divinyl benzene, an organic acid, and an organic acid anhydride may be used.

For performing effective cross-linking and securing desired physical properties, it may be advantageous to use a specific type of cross-linking agent.

In one example, as the cross-linking agent, an organic acid having two or more carboxyl groups, or an anhydride of the organic acid, or an organic compound having two or more aldehyde groups (formyl groups or aldehyde groups) may be applied.

The organic acid, anhydride of the organic acid, or organic compound may be composed of only carbon, oxygen, and hydrogen.

The type of the usable organic acid as the cross-linking agent is not particularly limited, but an organic acid with a molecular weight of about 90 g/mol to 300 g/mol, or about 100 g/mol to 250 g/mol may be used. The organic acid may have 2 to 10, 2 to 9, 2 to 8, 2 to 7, 2 to 6, 2 to 5, 2 to 4 or 2 to 3, or 2 or 3 carboxyl groups.

Such an organic acid may be exemplified by citric acid, succinic acid, pimelic acid, or adipic acid, and the like, but is not limited thereto.

The organic acid or its anhydride may be used as the cross-linking agent.

The type of the usable organic compound having two or more aldehyde groups as the cross-linking agent is not particularly limited, but an organic compound with a molar mass of about 90 g/mol to 200 g/mol, or about 90 g/mol to 150 g/mol may be used. The organic compound may have 2 to 10, 2 to 9, 2 to 8, 2 to 7, 2 to 6, 2 to 5, 2 to 4 or 2 to 3, or 2 or 3 aldehyde groups.

Such an organic compound may be exemplified by glutaraldehyde, and the like, but is not limited thereto.

In one example, an oxidized polysaccharide (including an oxidized disaccharide) may be applied as the cross-linking agent. In this instance, the definition of the polysaccharide is as described above. When the polysaccharide component is oxidized, the hydroxy group included in the polysaccharide is first transformed into a carbonyl group to form the aldehyde group, and as further oxidation proceeds, the carboxyl group can be generated. Since the aldehyde group or carboxyl group can participate in the cross-linking reaction, it can be used as the cross-linking agent. The method of oxidizing the polysaccharide is known.

The applicable oxidized polysaccharide may be exemplified by oxidized starch, oxidized dextrin, oxidized chitosan, oxidized chitin, oxidized sucrose, and/or oxidized maltose, and the like, but is not limited thereto.

As the oxidized polysaccharide, for example, a component having a molar mass in a predetermined range while having 2 to 10, 2 to 9, 2 to 8, 2 to 7, 2 to 6, 2 to 5, 2 to 4, or 2 to 3, or 2 or 3 aldehyde groups (formyl groups or aldehyde groups) and/or carboxyl groups may be used.

In another example, the lower limit of the ratio of the aldehyde group and/or carboxyl group per mole of the oxidized polysaccharide in the oxidized polysaccharide may be 0.01 mol, 0.05 mol, 0.1 mol, 0.15 mol, 0.2 mol, 0.25 mol, or 0.3 mol or so, and the upper limit thereof may be 0.9 mol, 0.85 mol, 0.8 mol, 0.75 mol, 0.7 mol, 0.65 mol, 0.6 mol, 0.55 mol, 0.5 mol, 0.45 mol, 0.4 mol, 0.35 mol, or 0.3 mol or so. The molar ratio of aldehyde group and/or carboxyl group per mole of the oxidized polysaccharide may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. The ratio can be confirmed through $^1$H NMR analysis of the oxidized polysaccharide. That is, the ratio can be confirmed because the carboxyl group and/or aldehyde group present in the polysaccharide can be quantified through the $^1$H NMR analysis. In this way, the method of quantifying functional groups through $^1$H NMR analysis is known.

The lower limit of the molar mass of the oxidized polysaccharide may be 100 g/mol, 150 g/mol, 200 g/mol, 250 g/mol, or 300 g/mol or so, and the upper limit thereof may be 1000 g/mol, 950 g/mol, 900 g/mol, 850 g/mol, 800 g/mol, 750 g/mol, 700 g/mol, 650 g/mol, 600 g/mol, 550 g/mol, 500 g/mol, 450 g/mol, 400 g/mol, or 350 g/mol or so. The molar mass may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. Usually, disaccharides among polysaccharides exhibit molar masses in the above range.

The polymer material treated with the such a cross-linking agent can satisfy the desired absorption properties and biodegradability degree while exhibiting appropriate gel strength.

In the polymer material, the lower limit of the weight ratio of the cross-linking agent relative to 100 parts by weight of the self-cross-linked polysaccharide component may be 0.01 parts by weight, 0.05 parts by weight, 0.1 parts by weight, 0.5 parts by weight, 1 part by weight, 1.5 parts by weight, 2 parts by weight, 2.5 parts by weight, 3 parts by weight, 3.5 parts by weight, 4 parts by weight, 4.5 parts by weight, or 5 parts by weight or so, and the upper limit thereof may be 20 parts by weight, 18 parts by weight, 16 parts by weight, 14 parts by weight, 12 parts by weight, 10 parts by weight, 8 parts by weight, or 6 parts by weight or so. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. Usually, disaccharides among polysaccharides exhibit molar masses in the above range.

Under such a ratio, the polymer material can satisfy the desired absorption properties and biodegradability degree while exhibiting appropriate gel strength.

The polymer material may comprise the polysaccharide component (polysaccharide component self-cross-linked and bound with the cross-linking agent) as described above, and may further comprise other components if necessary.

In one example, the lower limit of the ratio of the polysaccharide component (polysaccharide component self-cross-linked and bound with the cross-linking agent) in the polymeric material may be 55 wt%, 60 wt%, 65 wt%, 70 wt%, 75 wt%, 80 wt%, 85 wt%, 90 wt%, 92 wt%, 94 wt%, 96 wt%, or 98 wt% or so, and the upper limit thereof may be 100 wt%, 98 wt%, 96 wt%, 94 wt%, 92 wt%, or 90 wt% or so. The ratio may be less than any one of the above-described upper limits, or within a range of less than or equal to, or less than any one of the above-described upper limits while being more than or equal to, or more than any one of the above-described lower limits.

The ratio of the polysaccharide component in the polymer material is not particularly limited, but the higher the ratio, the greater the biodegradability of the polymer material. However, in the case of absorbent materials applying conventional polysaccharide components, if the ratio of polysaccharide components is excessively increased in consideration of biodegradability, there is a problem that the absorption capacity is reduced. However, in the present application, the desired absorption capacity can be stably achieved while maintaining the ratio of polysaccharide components at a high level.

Since the polymer material exhibits excellent absorbency and biodegradability simultaneously, it may be used for various applications.

For example, the polymer material may be used as sanitary products such as diapers or sanitary napkins, or

absorbent materials applied to other applications requiring absorption. If necessary, further cross-linking, surface treatment, or physical grinding processes may also be performed on the polymer material to increase the efficiency for use as the sanitary products or absorbent materials.

Therefore, the present application relates to an absorbent material or sanitary product (e.g., diapers, sanitary napkins, etc.) comprising the polymer material.

The specific method of forming the absorbent material or sanitary product by applying the polymer material is not particularly limited, and for example, the method of forming the absorbent material or sanitary product by applying the existing SAP may be used in the same manner.

## Advantageous Effect

The present application can provide a polymer material with excellent biodegradability and absorption capacity, and a use thereof.

## Description of Drawings

Figures 1 to 3 are $^1$H NMR spectra of materials obtained in Preparation Examples.

## Mode for Invention

Hereinafter, the present application will be described in detail below through examples and comparative examples, but the scope of the present application is not limited by the following examples.

### 1. Evaluation of centrifuge retention capacity (CRC)

Centrifuge retention capacity (CRC) was measured according to EDANA (European Disposables and Nonwovens Association) WSP 241.3. About 0.2 g ($W_0$) of the obtained polymer material was placed in a non-woven bag, sealed, and then submerged in a physiological saline solution. As the physiological saline solution, an aqueous NaCl solution with a concentration of 0.9 wt% was used. The state was maintained for 30 minutes or so, water was removed from the bag for 3 minutes under a condition of 250 G using a centrifuge, and the mass (g, $W_2$) of the bag was measured.

The same operation was performed on the same non-woven bag containing no polymer material, and the mass (g, $W_1$) was measured.

The CRC (g/g) was calculated by substituting the measurement results into Equation A below.

The evaluation was conducted under constant temperature and humidity conditions (23 ± 1°C, relative humidity: 50 ± 10%).

[Equation A]

$$\text{CRC (g/g)} = \{[W_2(g) - W_1(g)]/W_0(g)\} - 1$$

### 2. Evaluation of absorption capacity under pressure (AUP)

Absorption capacity under pressure (AUP, 0.7 psi) of a polymer material was measured according to the standard of EDANA (European Disposables and Nonwovens Association) WSP 242.3. A 400-mesh stainless steel wire net was mounted on the bottom of a plastic cylinder with an inner diameter of 60 mm or so. Under conditions of a temperature of 23 ± 2°C and a relative humidity of 50%, about 0.90 g ($W_0$) of the polymer material was uniformly sprayed on the wire net, and a piston capable of further uniformly imparting a load of about 0.7 psi was installed thereon to manufacture a measuring device. As the piston, a piston with an outer diameter slightly smaller than 60 mm was used, which was installed so that it could move up and down without forming a gap with the inner wall of the cylinder. The weight (unit: g) ($W_3$) of the measuring device was measured.

A glass filter with a diameter of 90 mm or so and a thickness of 5 mm or so was placed on the inside of a petro dish with a diameter of about 150 mm, and a physiological saline solution (NaCl aqueous solution with a concentration of 0.9 wt%) was applied to be at the same level as the upper surface of the glass filter. One sheet of filter paper with a diameter of 90 mm or so was placed thereon. The measuring device was placed on the filter paper and the physiological saline solution was absorbed for 1 hour under a load of 0.7 psi. After 1 hour, the measuring device was lifted, and the weight $W_4$ (g) was measured.

The absorption capacity under pressure (AUP) (g/g) was calculated by substituting the respective weights as measured into Equation B below.

[Equation B]

$$AUP\ (g/g) = [W_4(g) - W_3(g)]/W_0(g)$$

**3. Measurement of biodegradability degree**

A biodegradability degree was measured in the manner specified in ISO 14855-1 (2005) standard. The above standard is a method of measuring an aerobic biodegradability degree of plastic materials under a composting condition, which is a method that the biodegradability degree of a polymer material is calculated by quantifying the amount of carbon dioxide emitted by microorganisms metabolizing the relevant material. As the polymer material was applied to the composting condition according to the standard, the biodegradability degree was measured for 6 months, and the biodegradability degree was obtained as the ratio of the theoretical carbon dioxide generation amount and the actual carbon dioxide generation amount of the material. Here, the theoretical carbon dioxide generation amount and biodegradability degree are obtained according to the following equations C and D, respectively.

$$\text{Theoretical carbon dioxide generation amount (ThCO2, g/container)} = MTOT \times CTOT \times (44/12) \qquad \text{[Equation C]}$$

In Equation C, MTOT is the amount (g) of total dry solid content in the test material (polymer material) added to the compost at the start of the measurement, and CTOT means the ratio (g/g) of organic carbon contained in the total dry solid content of the test material.

[Equation D]

$$\text{Biodegradability degree (\%)} = [\{(CO2)T-(CO2)B\}/ThCO2] \times 100$$

In Equation D, (CO2)T is the accumulated amount (g/container) of carbon dioxide generated from the composting container containing the test material, (CO2)B is the average (g/container) of carbon dioxide accumulated amounts generated from the inoculum source container, and ThCO2 is the theoretical carbon dioxide generation amount confirmed in Equation C above.

**4. Molecular weight measurement of polysaccharide component**

A molecular weight (weight average molecular weight, unit: g/mol) of a polysaccharide component was evaluated in the following manner.

(1) Preparation of mobile phase

A mobile phase A was prepared by filtering 1000 mL of a 150 mM $NaNO_3$ aqueous solution containing 0.02 wt% of $NaN_3$ using a solvent clarification system (Millipore Millisolve Kit, MilliporeSigma).

(2) Preparation of sample solution

A sample intended to be measured was collected in an amount of 25 mg, and mixed with 5 mL of a 150 mM $NaNO_3$ aqueous solution containing 0.02 wt% of $NaN_3$, and then a sample solution was prepared by heating the mixture at 80°C for 20 hours, and then filtering it with a 0.4μm Nylon Syringe Filter.

(3) GPC (Gel Permeation Chromatography)/MALS (Multi-Anglue Light Scattering Detection) conditions

The molecular weight was evaluated using the sample solution and the mobile phase A in the following manner.
Measuring instrument: Agilent GPC (Agilent 1200 series, U.S.)

Stationary phase: connecting Shodex OH-Pak 804 column and Shodex OH-Pak 80 column

Mobile phase: A; 0.02% $NaN_3$, 150 mM $NaNO_3$ aqueous solution = 100 (v/v %)

Flow rate: 0.4 mL/min

Stationary phase temperature: 25°C

Injected amount: 100 μl (0.45 μm filtered)

Analysis time: 120 minutes

**Preparation Example 1.**

A polysaccharide (modified chitosan) (Compound A) containing a modified monosaccharide unitary body of Formula A below was prepared in the following manner. The modified monosaccharide unitary body of Formula A below is a monosaccharide unitary body into which a maleic acid group is introduced.

[Formula A]

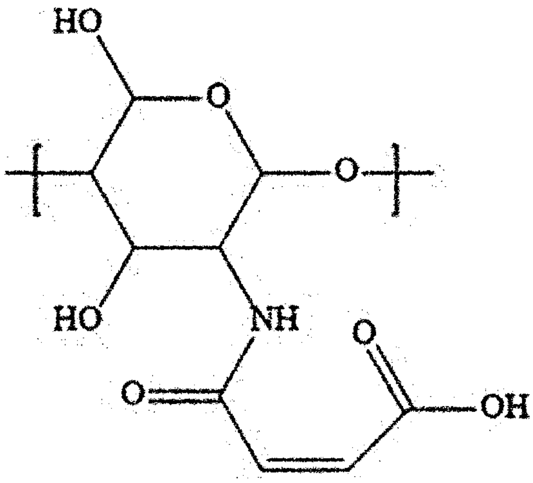

15 g of chitosan and 300 mL of DMSO (dimethyl sulfoxide) were added to a 500 mL RBF (Round Bottom Flask), and stirred at 65°C for 30 minutes to be gelatinized. About 30 g of maleic anhydride was added to the gelatinized chitosan, and reacted at 65°C for about 3 hours. After completion of the reaction, the temperature was lowered to room temperature (about 25°C) or so, and acetone was added thereto to produce a precipitate. The precipitate was recovered, and dried in a vacuum drying oven at 40°C for one day to obtain a solid target product (Compound A).

A substitution degree of the obtained target product (Compound A) can be obtained through a $^1H$ NMR analysis. The $^1H$ NMR analysis is performed at room temperature (about 25°C) using a $^1H$ NMR spectrometer including a Varian Unity Inova (500 MHz) spectrometer with a triple resonance 5 mm probe. In the $^1H$ NMR analysis, Bruker's Avance Neo instrument was used.

50 mg of the obtained solid target product (Compound A) and 200 mg of 30% DCl in D2O solution are mixed, and stirred at 50°C for 1 hour or so to induce a hydrolysis reaction, and the $^1H$ NMR analysis can be performed.

The substitution degree of the maleic acid group into the chitosan was confirmed by $^1H$ NMR analysis. Figure 1 is a $^1H$ NMR spectrum of the target product. Referring to Figure 1, peaks are identified at 6.3 ppm and 5.8 ppm corresponding to the vinyl group of the maleic acid group, through which it can be confirmed that the maleic acid group has been introduced into the chitosan. Based on the integral ratio of the peaks, the substitution degree of the maleic acid group (-OCOCH=CHCOOH) in the maleated chitosan can be known, and the substitution degree obtained in this way was about 0.94 or so.

**Preparation Example 2.**

A polysaccharide (modified starch) (Compound B) containing a modified monosaccharide unitary body of Formula B below was prepared in the following manner. The modified monosaccharide unitary body of Formula B below is a monosaccharide unitary body into which a maleic acid group is introduced.

[Formula B]

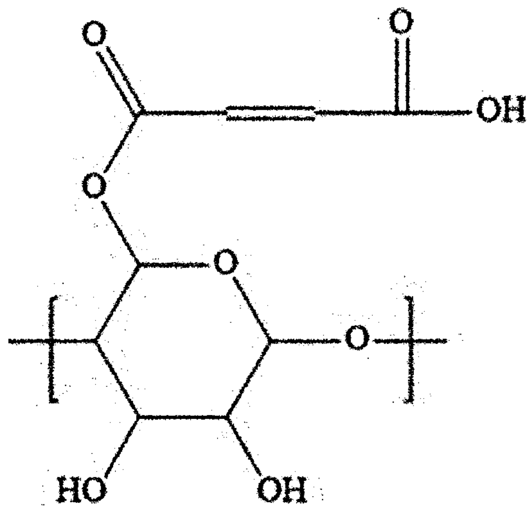

15 g of starch and 50 mL of DMSO (dimethyl sulfoxide) were added to a 500 mL RBF (Round Bottom Flask), and stirred at 65°C for 2 hours to be gelatinized. As the starch, potato starch was used. About 30 g of maleic anhydride was added to the gelatinized starch, and reacted at 65°C for 3 hours or so. After completion of the reaction, the temperature was lowered to room temperature, and acetone was added thereto to generate a precipitate. The precipitate was recovered, and dried in a vacuum drying oven at 40°C for one day to obtain a solid target product (Compound B).

50 mg of the obtained solid target product (Compound B) and 200 mg of 30% DCl in D2O solution are mixed, and stirred at 50°C for 1 hour or so to induce a hydrolysis reaction, and then $^1H$ NMR analysis can be performed.

The substitution degree of the target product (Compound B) was confirmed through $^1H$ NMR analysis in the same manner as in Preparation Example 1, and as a result, the substitution degree was approximately 0.8 or so. Figure 2 is $^1H$ NMR analysis results of the target product.

**Preparation Example 3.**

An oxidized polysaccharide (oxidized disaccharide) (oxidized maltose) was prepared in the following manner. 20 g of maltose was dissolved in 100 mL of distilled water in a 500 mL RBF (Round Bottom Flask). Light was blocked with a foil, and about 0.5 to 0.75 equivalents of $NaIO_4$ was introduced thereto, and stirred at room temperature (about 25°C) for about 24 hours. Barium acetate was further introduced thereto, and stirred, and then a solution filtered through a filter was dried to obtain oxidized maltose. Figure 3 is $^1H$ NMR analysis results of the target product (oxidized maltose). The degree of aldehyde group generation in the oxidized maltose can be confirmed by examining the $^1H$ NMR analysis results. In the $^1H$ NMR analysis results, the integration value of the peak (peak corresponding to the aldehyde group) identified within the range of 8.0 ppm to 8.5 ppm is set to 1, and the integration values of other peaks are set based on this. Afterwards, the integration value of the peak identified within the range of 3.0 ppm to 4.0 ppm is divided by 14 to confirm the value. The peak identified within the range of 3.0 ppm to 4.0 ppm is a peak derived from the ring structure of oxidized polysaccharide (oxidized maltose), and thus 14 is a value related to the value obtained by dividing it by 14 and the number of hydrogen atoms present in the ring structure. Therefore, through comparison of the values obtained by the integration value of the peak (peak corresponding to the aldehyde group) identified within the range of 8.0 ppm to 8.5 ppm and the integration value of the peak identified within the range of 3.0 ppm to 4.0 ppm divided by 14, the ratio of aldehyde groups contained in oxidized maltose can be confirmed. As a result of confirmation in this manner, the oxidized maltose contained about 0.3 mole of aldehyde group per mole.

**Example 1**

A self-cross-linked polysaccharide component was prepared by self-cross-linking CMC (carboxymethyl cellulose). When an aqueous solution with a concentration of 1 wt% was prepared with the CMC, the CMC in which the viscosity of the aqueous solution was about 1600 cP to 1700 cP, and the degree of substitution (DS) was about 0.7 to 0.8 was used as the CMC (carboxymethyl cellulose). Here, the viscosity of the aqueous solution was measured using a Brookfield viscometer-DV2T device, where a V-74 spindle was selected as the spindle, the speed was set to 50 rpm, and the measurement temperature was set to 25°C. In addition, the degree of substitution (DS) is the degree of substituting the carboxyl groups present in the CMC, which was obtained through $^1H$ NMR analysis results based on the cases of

Preparation Examples 1 and 2. 20 g of the CMC was dissolved in 800 mL of distilled water, spread thinly on a tray, and then dried in an oven at about 40°C. The pH of the mixture of CMC and distilled water was adjusted to 10.5 or so by adding NaOH aqueous solution of about 1N. After the drying, the CMC was heated at 120°C for 3 hours to prepare a self-cross-linked polysaccharide component, and the polysaccharide component was pulverized and classified to obtain a material having a particle size of about 300 μm to 600 μm.

Surface cross-linking was performed on the obtained material. The surface cross-linking was performed using a surface cross-linking solution prepared by dissolving 0.18 g of the compound (oxidized maltose) of Preparation Example 3 together with 0.018 g of $AlCl_3$ in a solution of 0.6 g of acetone and 0.6 g of water. 3.6 g of the material pulverized and classified to have a particle size of about 300 μm to 600 μm was placed on an aluminum dish, and the cross-linking solution was sprayed evenly. The mixture was mixed until the cross-linking agent was sufficiently mixed, and then heated at 120°C for 30 minutes to obtain a polymer material comprising the self-cross-linked polysaccharide component and the cross-linking agent bound to the component. The obtained material was further pulverized and classified as necessary.

**Example 2.**

A polymer material was prepared in the same manner as in Example 1, except that as the CMC, the CMC in which the viscosity of the aqueous solution containing 1 wt% of the CMC was 9000 cP to 10000 cP or so, and the degree of substitution (DS) was 0.65 to 0.75 was used. The methods of measuring the viscosity and substitution degree are the same as in Example 1.

**Example 3.**

A polymer material was prepared in the same manner as in Example 1, except that the target product (Compound A) of Preparation Example 1 was used instead of CMC.

**Example 4.**

A polymer material was prepared in the same manner as in Example 1, except that the target product (Compound B) of Preparation Example 2 was used instead of CMC.

**Comparative Example 1**

A polymer material was prepared in the same manner as in Example 4, but the polymer material was prepared without performing a surface cross-linking process after self-cross-linking.

The physical property measurement results for the polymer materials are shown in Table 1 below.

[Table 1]

| | Example | | | | Comparative Example |
|---|---|---|---|---|---|
| | 1 | 2 | 3 | 4 | 1 |
| CRC (g/g) | 40.2 | 32.6 | 28.1 | 26.4 | 35 |
| AUP (g/g) | 13.2 | 19.9 | 12.8 | 8.9 | 4.5 |
| Biodegradability degree (%) | 89 | 90 | 87 | 95 | 94 |

**Claims**

**1.** A polymer material comprising:

a self-cross-linked polysaccharide component; and
a cross-linking agent bound with the self-cross-linked polysaccharide component.

**2.** The polymer material according to claim 1, wherein the self-cross-linked polysaccharide component is in a particle shape and the cross-linking agent is bound to the surface of the particle shape.

**3.** The polymer material according to claim 1, having a centrifuge retention capacity of 10 g/g or more according to

EDANA (European Disposables and Nonwovens Association) method WSP 241.3.

**4.** The polymer material according to claim 1, having an absorption capacity under pressure at 0.7 psi of 3 g/g or more according to EDANA (European Disposables and Nonwovens Association) method WSP 242.3.

**5.** The polymer material according to claim 1, having a biodegradability degree of 50% or more.

**6.** The polymer material according to claim 1, wherein polysaccharide is an acidic polysaccharide having a substitution degree in a range of 0.4 to 2.5.

**7.** The polymer material according to claim 1, wherein the self-cross-linked polysaccharide component comprises polymer chains containing monosaccharide units linked by glycosidic bonds, and bonds of Formula 1 below linking the polymer chains:

[Formula 1]

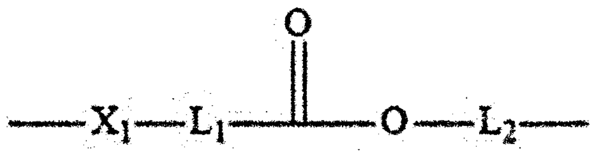

wherein, $X_1$ is an oxygen atom or $NR_{11}$, where $R_{11}$ is a hydrogen atom, an alkyl group or an alkylcarbonyl group, and $L_1$ is an alkylene group, an alkylidene group, or a bond of Formula 2 below, and $L_2$ is represented by a single bond or $-(CH_2)-O-$.

[Formula 2]

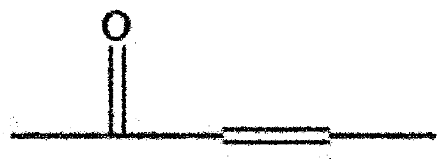

**8.** The polymer material according to claim 7, wherein the monosaccharide unit has a ring structure including carbon atoms and oxygen atoms as ring constituent atoms, and the bond of Formula 1 is directly connected to the carbon atom of the ring structure, or is connected via a methylene group.

**9.** The polymer material according to claim 1, wherein the polysaccharide component contains a unit represented by Formula 3 below:

[Formula 3]

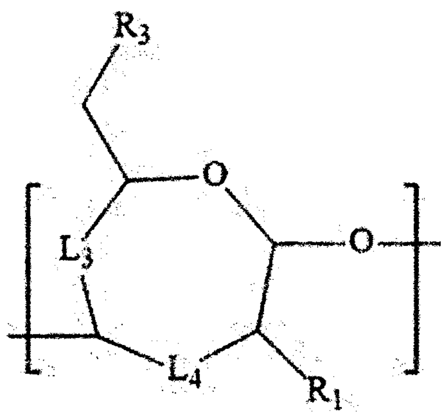

wherein, $R_1$ is a hydroxy group, an amino group, $-L_5-C(=O)-OH$, $-L_5-C(=O)-O^-$, or a functional group of Formula 4 below, $R_3$ is a hydroxy group, $-L_5-C(=O)-OH$, $-L_5-C(=O)-O^-$ or a functional group of Formula 4 below, and one of $L_3$ and $L_4$ is a single bond, the other is $CHR_2$, where $R_2$ is a hydroxy group, $-L_5-C(=O)-OH$, $-L_5-C(=O)-O^-$ or a functional group of Formula 4 below, and $L_5$ is an alkylene group or an alkylidene group, but any one of $R_1$ to $R_3$ is the oxygen atom (excluding the oxygen atom present in the carbonyl group) of the bond of Formula 1 above.

[Formula 4]

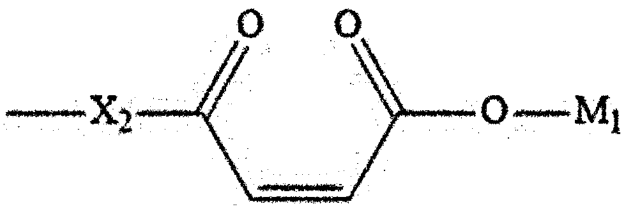

wherein, $X_2$ is an oxygen atom or $NR_{11}$, where $R_{11}$ is a hydrogen atom, an alkyl group or an alkylcarbonyl group, $M_1$ is hydrogen or a metal, and when $M_1$ is the metal, the bond of $O-M_1$ above is an ionic bond.

**10.** The polymer material according to claim 1, wherein the cross-linking agent is one or more selected from the group consisting of a polyfunctional epoxy compound, an epoxy silane compound, an amino silane compound, epichlorohydrin, formaldehyde, glutaraldehyde, oxidized sucrose, an acyl chloride, a carbonate, a diamine, a diol, carbon disulfide, phosphoryl chloride, divinyl benzene, an organic acid, and an organic acid anhydride.

**11.** The polymer material according to claim 1, wherein the cross-linking agent is an organic acid having two or more carboxyl groups, an anhydride of the organic acid, or an organic compound having two or more aldehyde groups.

**12.** The polymer material according to claim 1, wherein the cross-linking agent is an oxidized polysaccharide.

**13.** The polymer material according to claim 1, wherein the cross-linking agent is one or more selected from the group consisting of oxidized starch, oxidized dextrin, oxidized chitosan, oxidized chitin, oxidized sucrose, and oxidized maltose.

**14.** The polymer material according to claim 12, wherein the oxidized polysaccharide contains aldehyde groups in a range of 0.01 moles to 0.9 moles per mole.

**15.** The polymer material according to claim 12, wherein the oxidized polysaccharide has a molar mass in a range of 100 g/mol to 1000 g/mol.

**16.** The polymer material according to claim 1, comprising 0.01 to 20 parts by weight of the cross-linking agent relative to 100 parts by weight of the self-cross-linked polysaccharide component.

**17.** An absorbent material comprising the polymer material of any one of claims 1 to 16.

**18.** A sanitary article comprising the polymer material of any one of claims 1 to 16.

[Figure 1]

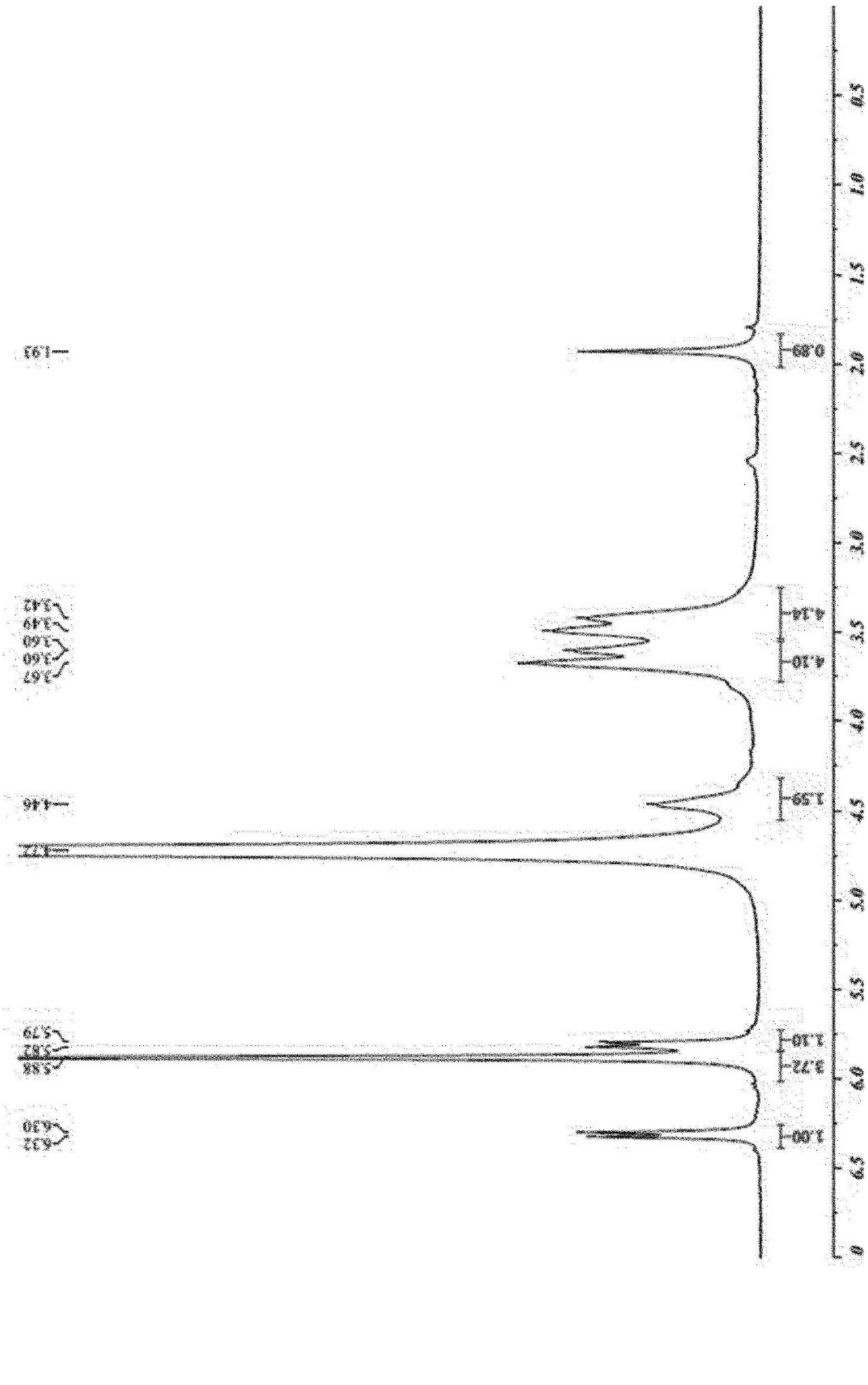

[Figure 2]

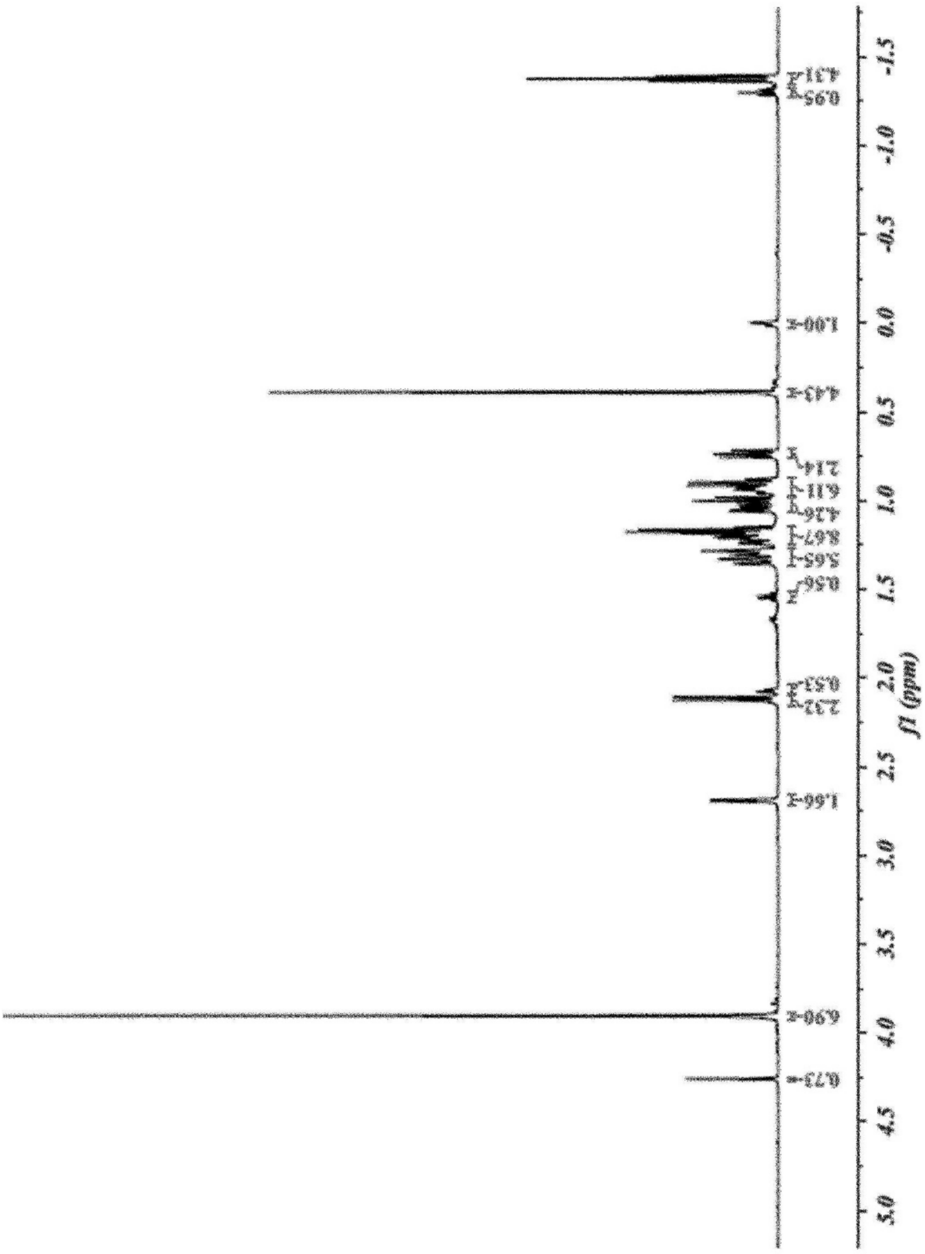

[Figure 3]

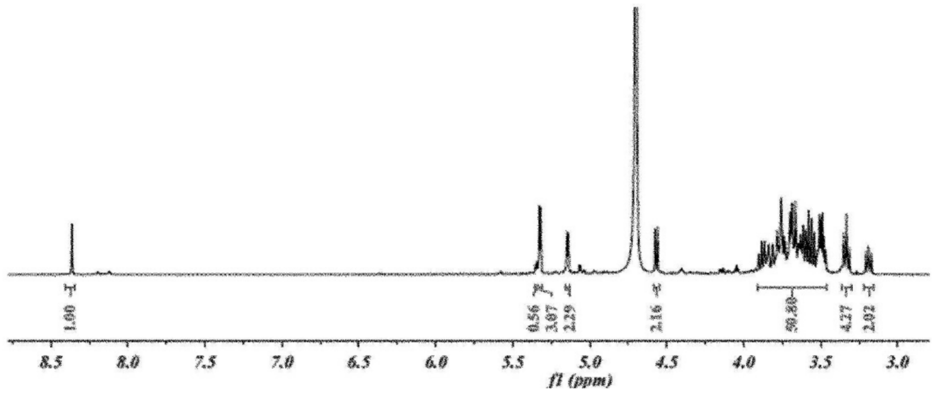
1.00
0.56
3.07
2.29
2.16
50.80
4.27
2.02
8.5
8.0
7.5
7.0
6.5
6.0
5.5
5.0
4.5
4.0
3.5
3.0
f1 (ppm)

**INTERNATIONAL SEARCH REPORT**

International application No. **PCT/KR2024/000879**

**A. CLASSIFICATION OF SUBJECT MATTER**

**C08J 3/24**(2006.01)i; **C08J 3/12**(2006.01)i; **C08B 15/00**(2006.01)i; **C08L 1/28**(2006.01)i; **C08K 5/00**(2006.01)i; **C08K 5/092**(2006.01)i; **C08K 5/07**(2006.01)i; **A61L 15/28**(2006.01)i

According to International Patent Classification (IPC) or to both national classification and IPC

**B. FIELDS SEARCHED**

Minimum documentation searched (classification system followed by classification symbols)

C08J 3/24(2006.01); A61K 31/715(2006.01); C07H 5/06(2006.01); C08B 31/10(2006.01); C08B 37/00(2006.01); C08J 3/28(2006.01); C08L 5/00(2006.01); C08L 89/00(2006.01); G01N 21/3577(2014.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 자가-가교(self-crosslinking), 다당류(polysaccharide), 전분(starch), 가교제(crosslinking agent)

**C. DOCUMENTS CONSIDERED TO BE RELEVANT**

| Category* | Citation of document, with indication, where appropriate, of the relevant passages | Relevant to claim No. |
|---|---|---|
| X | KR 10-1242010 B1 (ARCHER-DANIELS-MIDLAND COMPANY) 13 March 2013 (2013-03-13) See claims 1, 7, 11 and 12; and paragraphs [0119], [0120], [0121], [0134] and [0200]-[0202]. | 1-11,16-18 |
| Y | | 12-15 |
| Y | KR 10-2001-0105311 A (SCA HYGIENE PRODUCTS ZEIST B.V.) 28 November 2001 (2001-11-28) See abstract; claims 1 and 12-14; and page 6. | 12-15 |
| A | JP 6055466 B2 (THE UNIVERSITY OF TOKYO) 27 December 2016 (2016-12-27) See claims 1-21. | 1-18 |
| A | JP 2008-111027 A (JAPAN ATOMIC ENERGY AGENCY) 15 May 2008 (2008-05-15) See claims 1-10. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 April 2024** | **29 April 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2024/000879**

**C. DOCUMENTS CONSIDERED TO BE RELEVANT**

| Category* | Citation of document, with indication, where appropriate, of the relevant passages | Relevant to claim No. |
|---|---|---|
| A | US 2008-0300218 A1 (ABE, Y. et al.) 04 December 2008 (2008-12-04)<br>See entire document. | 1-18 |

Form PCT/ISA/210 (second sheet) (July 2022)

**INTERNATIONAL SEARCH REPORT**
**Information on patent family members**

International application No.
**PCT/KR2024/000879**

| Patent document cited in search report | Publication date (day/month/year) | Patent family member(s) | Publication date (day/month/year) |
|---|---|---|---|
| KR 10-1242010 B1 | 13 March 2013 | AU 2007-302586 A1 | 03 April 2008 |
| | | AU 2007-302586 B2 | 27 June 2013 |
| | | BR PI0717171 A2 | 15 October 2013 |
| | | BR PI0717171 B1 | 17 October 2023 |
| | | CA 2664392 A1 | 03 April 2008 |
| | | CN 101541836 A | 23 September 2009 |
| | | CN 101541836 B | 08 April 2015 |
| | | CN 104774275 A | 15 July 2015 |
| | | EP 2066699 A1 | 10 June 2009 |
| | | EP 2066699 B1 | 12 February 2020 |
| | | JP 2010-504414 A | 12 February 2010 |
| | | JP 2013-253262 A | 19 December 2013 |
| | | JP 5765884 B2 | 19 August 2015 |
| | | JP 5947768 B2 | 06 July 2016 |
| | | KR 10-1329658 B1 | 14 November 2013 |
| | | MX 2009003252 A | 02 November 2009 |
| | | MX 339603 B | 31 May 2016 |
| | | US 2008-0177057 A1 | 24 July 2008 |
| | | US 2013-0296548 A1 | 07 November 2013 |
| | | US 8461129 B2 | 11 June 2013 |
| | | WO 2008-037082 A1 | 03 April 2008 |
| KR 10-2001-0105311 A | 28 November 2001 | AU 2000-18975 A1 | 03 July 2000 |
| | | AU 2000-18975 B2 | 27 November 2003 |
| | | BR 9916235 A | 04 September 2001 |
| | | CA 2356849 A1 | 22 June 2000 |
| | | EP 1140229 A1 | 10 October 2001 |
| | | EP 1140229 B1 | 06 October 2010 |
| | | JP 2002-532573 A | 02 October 2002 |
| | | MX PA01006098 A | 27 March 2002 |
| | | NZ 512254 A | 28 November 2003 |
| | | PL 348821 A1 | 17 June 2002 |
| | | SK 7982001 A3 | 05 February 2002 |
| | | TN SN99243 A1 | 31 December 2001 |
| | | US 2004-0236016 A1 | 25 November 2004 |
| | | US 6765042 B1 | 20 July 2004 |
| | | WO 00-35504 A1 | 22 June 2000 |
| | | ZA 200104559 B | 04 June 2002 |
| JP 6055466 B2 | 27 December 2016 | WO 2013-176239 A1 | 28 November 2013 |
| JP 2008-111027 A | 15 May 2008 | None | |
| US 2008-0300218 A1 | 04 December 2008 | EP 1595892 A1 | 16 November 2005 |
| | | EP 1595892 A4 | 20 June 2007 |
| | | JP 4638817 B2 | 23 February 2011 |
| | | US 2006-0178339 A1 | 10 August 2006 |
| | | US 7485719 B2 | 03 February 2009 |
| | | WO 2004-081055 A1 | 23 September 2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230008024